Europäisches Patentamt

European Patent Office

Office européen des brevets

(11) Publication number: **0 471 954 A2**

# EUROPEAN PATENT APPLICATION

(21) Application number: **91110851.2**

(22) Date of filing: **01.07.91**

(51) Int. Cl.5: **A61K 39/10, A61K 39/385**

(30) Priority: **13.08.90 US 565162**

(43) Date of publication of application:
**26.02.92 Bulletin 92/09**

(84) Designated Contracting States:
**AT BE CH DE DK ES FR GB GR IT LI NL SE**

(71) Applicant: **AMERICAN CYANAMID COMPANY**
**1937 West Main Street P.O. Box 60**
**Stamford Connecticut 06904-0060(US)**

(72) Inventor: **Kimura, Alan**
**9856 N.W. 52 Terrace**
**Miami, Florida 33178(US)**
Inventor: **Beurret, Michel**
**721 1/2 University Avenue**
**Rochester, New York(US)**
Inventor: **Cowell, James Leo**
**37 Sugarmills Circle**
**Fairport, New York 14450(US)**

(74) Representative: **Wächtershäuser, Günter, Dr.**
**Tal 29**
**W-8000 München 2(DE)**

(54) **Immunogenic conjugates of nontoxic oligosaccharide derived from bordetella pertussis lipooligosaccharide.**

(57) Non-toxic oligosaccharides derived from Bordetella pertussis lipooligosaccharide and conjugates thereof wherein the oligosaccharides are bound to a carrier protein such as filamentous hemagglutinin of Bordetella pertussis, vaccine compositions containing the conjugates and methods of eliciting an immune response against Bordetella pertussis comprising administering such conjugates with a pharmaceutically acceptable vehicle to a warm-blooded animal.

EP 0 471 954 A2

I. Field of the Invention

This invention is directed to the field of novel vaccine compositions and methods of provoking an immunogenic response in warm-blooded animals against infections caused by Bordetella pertussis. More particularly, this invention relates to novel nontoxic oligosaccharides derived from Bordetella pertussis lipooligosaccharide and conjugates wherein such oligosaccharides are linked to a carrier protein, and vaccine compositions containing the conjugates. The present invention also relates to methods of preparing such oligosaccharides and conjugates thereof.

II. Background of the Invention

A. Pertussis and Pertussis Vaccines

The bacterium Bordetella pertussis is a respiratory tract pathogen which is the causative agent of the serious infectious disease known as pertussis or whooping cough. The organism is a small, Gram-negative bacillus first cultured in 1906. Before the advent of killed whole cell vaccines, which were licensed in the United States in the early 1940s, the mortality in infants from pertussis reached five per 1000 (Mortimer, et al., Rev. Infect. Dis. 1, 927-32 (1979)). While the current inactivated whole-cell vaccine has decreased the incidence of the disease (Fine et al., Reflections on the Efficacy of Pertussis Vaccines, Rev. Infect. Dis. 9, 866-883 (1987)) it is generally accepted that reactogenicity of the DTP vaccine is significant and that the whole cell pertussis component plays a role. Questions have been raised as to possible serious neurological consequences after immunization with DTP (see for instance: Murphy, M.D., Rasnack, J., Dickson, H.D., Deitch, M. and Brunell, P.A., "Evaluation of the Pertussis components of DTP vaccine", Pediatrics 71W:200-205 (1983); Stetler, H.C., Orenstein, W., Bart, K.J., Brink, E.W., Brennan, J.P. and Hinman, A., "History of convulsions and use of Pertussis vaccine", J. Pediatr. 104W:175-179 (1985)). However, more recent studies have been unable to show statistically significant linkage between vaccination with DTP and encephalopathy. (Cherry, JAMA 263 (12), 1679-1680 (1990) and Griffin et al., JAMA 263 (12), 1641-1645 (1990)).

Seizures and neurological problems associated with whole-cell vaccines, which contain the antigenic components necessary to elicit protective immunity, have intensified investigations into the pathogenic mechanisms of Bordetella pertussis to determine which antigenic components contribute to protective immunity and which are associated with undesirable effects.

Improved understanding of the biology of Bordetella pertussis has led to the development of acellular pertussis vaccines which contain antigenic components which confer immunity to disease without introducing irrelevant or undesired effects. Acellular pertussis vaccines are routinely used in Japan. Three antigenic fractions are regarded as being useful components of acellular vaccines; (1) lymphocytosis promoting factor (LPF), also known as pertussis toxin (PT), pertussigen, histamine sensitizing factor (HSF) or islets activating protein (IAP); (2) filamentous haemagglutinin (FHA) and (3) fimbrial agglutinogens (fimbriae) (See for example Weiss, A.A. and Hewlett, E.L., Ann. Rev. Microbiol., 40, 661-686 (1986)).

B. Bacterial Saccharides as Antigens in Vaccines

Bacterial saccharides such as bacterial capsular polysaccharides (CPS) are among the groups of antigens which have potential to be employed effectively in acellular vaccines. CPS are cell-surface antigens composed of repeating (oligo) saccharide units. It is known that purified capsular polysaccharides may be immunogenic in nature and can be used as vaccines against the corresponding localized and systemic infections. CPS, which may be produced by Gram-positive or Gram-negative bacteria, have been isolated and purified and used either alone or as a component in vaccines to confer protection against the systemic disease. Such protection is well established for the CPS of Haemophilus influenzae type b (Hib), Neisseria meningitis and Streptococcus pneumoniae. Unfortunately, however, most of these polymers are thymus independent antigens and are thus immunogenic only in adults and older children.

A second class of cell-surface, carbohydrate antigens of potential use in vaccines is produced by Gram-negative bacteria. Varying widely in structure and molecular weight, they are termed lipopolysaccharides (LPS) or lipooligosaccharides (LOS), known collectively as "endotoxins." With structural variations, all contain a toxic lipid moiety (lipid A) and an oligosaccharide core which may have a characteristic attached polysaccharide termed the 0-antigen, or 0-chain. The toxicity and strong immunogenicity of LPS/LOS are associated with the lipid A moiety. Removal of the lipid A moiety leads to detoxification, but also greatly reduces the immunogenicity of the LPS/LOS isolates.

The lipooligosaccharide (LOS) has been prepared from Bordetella pertussis endotoxin. (See, e.g., R.

Chaby and M. Caroff, Pathogenesis and Immunity in Pertussis, Chap. 12, pp. 247-271, John Wiley & Sons (1988)). Several investigators have described the occurrence of anti-LOS antibodies after infection with B. pertussis or after vaccination with killed whole cell B. pertussis vaccines, Shon Li et al., Infection and Immunity v. 56, p. 699-702 (1988). In addition, monoclonal antibodies specific to the pertussis lipopolysaccharide have been isolated and some of these antibodies demonstrated biological activity in an in vitro bactericidal assay, (P. Rondeau et al., Abstracts of ASM Annual Meeting, Abstract Number B-142, May 1990, p. 50).

However, the LOS of Bordetella pertussis has not heretofore been identified as a protective immunogen in animal models. In general, the prevailing view has been that the LOS of Bordetella pertussis does not elicit a protective immune response and does not play a role in clinical immunity. See for example, Robinson, A, Irons, L.I., and Ashworth, L.A.E., Vaccine 3:11-22 (1985) wherein it is stated that a specific protective role for LPS has not been established in any model of pertussis infection and that laboratory isolated LPS was found to be non-protective in mice. See also, E.A. Mortimer, "Pertussis and Pertussis Vaccine: 1990", in Adv. Pediatr. Infect. Dis. Vol. 5. Yearbook Medical Publishers, Inc., Chicago, pp 1-31 (1990). In addition, numerous adverse effects induced by some vaccine preparations have generally been attributed to the endotoxin and its removal has been considered desirable in order to improve the quality of vaccine preparations.

Thus, saccharide immunogens from B. pertussis LOS have not been extensively investigated as components in vaccine preparations.

SUMMARY OF THE INVENTION

This invention pertains to a novel nontoxic oligosaccharide derived from Bordetella pertussis lipooligosaccharide coupled to a carrier protein the conjugate of which is a useful component of an immunogenic Bordetella pertussis vaccine.

The novel oligosaccharide is coupled to a carrier protein such as filamentous hemagglutinin (FHA) derived from Bordetella pertussis, or CRM$_{197}$ (a non-toxic mutant of diphtheria toxin), or a portion thereof, wherein the antigen is not normally associated with the carrier protein.

The invention also pertains to a vaccine composition comprising the conjugate alone or in combination with other components of an acellular vaccine and a pharmaceutically acceptable vehicle.

The invention further pertains to a method for eliciting a protective immune response which comprises administering to a warm blooded animal an immunogenic amount of a vaccine composition comprising the immunogenic conjugate of the present invention in a pharmaceutically acceptable vehicle and an optional adjuvant.

A carrier protein such as FHA has several advantages in that it is nontoxic and of a relatively high molecular weight (200,000 KDa). In addition, the conjugation of a carrier protein such as FHA with a carbohydrate antigen from B. pertussis results in an immune response to both the coupled saccharide and the carrier protein thereby enhancing the protective immune response against B. pertussis.

Accordingly, the immunogenic conjugates of this invention comprise an oligosaccharide bound to a carrier protein, which carrier protein may be immunologically active, thereby providing an immunogenic conjugate which can induce or enhance an immune response to the oligosaccharide.

The oligosaccharides of this present invention may be prepared by treating the endotoxin of Bordetella pertussis with mild acid to cleave the glycosidic bond of 3-deoxy-2-octulosonic acid as shown by Osborn, M. J. Proc. Natl. Acad. Sci. 50, 499-506 (1963), thus separating the lipid A region from the water-soluble polysaccharide portion of the molecule. More recent work, Caroff et al., Infect. Immun. 54, 465-471 (1986), has shown that a complete severance of the lipid A region from the polysaccharide portion is best accomplished by incubation of Bordetella pertussis endotoxin in sodium acetate buffer at pH 4.5 containing SDS (1% w/v).

The conjugates of this invention can be prepared by any of the biologically compatible methods known in the art for coupling of antigens to carriers. The method of coupling is most preferably covalent whereby the antigen is bound directly to a protein carrier such an FHA of Bordetella pertussis. However, other means by which the antigen is conjugated to carrier proteins are included within the scope of this invention. Many such methods are currently available for coupling of antigens to protein carriers, see for example: Stowell and Lee (1980) "Neoglycoproteins" Adv. Carbohydr. Chem. Biochem. 37:225-281. Most methods create either amine or amide bonds but thioesters have also been formed. A particularly preferred method for coupling an antigen such as an oligosaccharide to an immunogenic carrier protein such as FHA is by reductive amination which has been described by Anderson, P.W., U.S. Patent No. 4,673,573, issued June 16, 1987 and U.S. Patent No. 4,761,283, issued August 2, 1988, the teachings of which are incorporated herein by reference.

The conjugates of the invention may be prepared by conventional glycoconjugation techniques, either through the interposition of short linker (spacer) chains or by direct coupling. For example, the conjugates may be prepared by first forming reducing end groups on the oligosaccharide and reacting these with amine groups of the carrier protein by reductive amination. Alternatively, the immunogenic conjugates of the invention may be prepared by direct coupling of the oligosaccharide by treatment with a carbodiimide forming a carboxylate intermediate which readily condenses with primary amino groups of the carrier protein.

The conjugates of this invention can be used to elicit an immune response to an antigen or saccharide in a warm-blooded animal such as a mammalian host. The method comprises administering to the animal an immunologically effective dose of the conjugate comprising an antigen bound to a protein carrier such as FHA.

The covalent conjugates of the invention may be formulated with a pharmaceutically acceptable vehicle and other optional immunogenic components to produce a vaccine which elicits antibody formation sufficient to confer protection against respiratory disease caused by Bordetella pertussis in the warm blooded animal.

DETAILED DESCRIPTION

A. Description of Oligosaccharide Component

1. Preparation of oligosaccharide from Bordetella pertussis Tohama I lipopolysaccharide.

The lipopolysaccharide (LPS) is purified from Bordetella pertussis Tohama I cells using the hot phenol-water method described by Johnson, K.G. and M. B. Perry, "Improved techniques for the preparation of bacterial lipopolysaccharides", Can. J. Microbiol. 22, 29-34 (1976).

The lyophilized LPS is hydrolyzed by heating in 1% acetic acid, frozen, thawed, centrifuged, and the supernatant extracted with chloroform:methanol followed by purification of the aqueous layer using gel filtration to give the Bordetella pertussis oligosaccharide (BPOS).

2. LAL Reactivity of B. pertussis Oligosaccharide Preparation

Young et al., (J. Chem. Invest. 51W:1790, 1972) have presented evidence that endotoxins activate an enzyme in LAL (Limulus amebocyte Lysate) which then reacts with a low molecular weight clottable protein to form a gel. In general, the LAL clotting activity parallels the pyrogenicity or endotoxin content of the sample. The LAL reactivity of B. pertussis oligosaccharide prepared as set forth above was compared to that of the whole lipooligosaccharide endotoxin from Tohama I cells in LAL clotting assay. The Pyrotell® Assay (Associates of Cape Cod, Inc., Falmouth, MA) for the detection and quantitation of endotoxins was used. As set forth in Table I, the results demonstrated that the oligosaccharide had much reduced reactivity than the LOS.

## Table 1

## Toxicity of <u>Bordetella</u> <u>pertussis</u> core oligosaccharide preparations.

|  | **Concentration resulting in** |  |
| --- | --- | --- |
| **Preparation** | <u>Limulus</u> **amebocyte lysate clotting** | **% activity** |
| **Tohama I LOS** | **0.0025** $\mu$**g/ml** | **100** |
| **Core oligosaccharide (preparation 1)** | **>100.0** $\mu$**g/ml** | **<0.0025** |
| **Core oligosaccharide (preparation 2)** | **12.5** $\mu$**g/ml** | **0.02** |

B. Description of Suitable Carrier Proteins

Suitable carrier proteins are those which are safe for administration to warm-blooded animals and effective as carriers. Safety considerations include the absence of primary toxicity and minimal risk of allergic reactions. Diphtheria and tetanus toxoids satisfy the safety aspect in that they are non-toxic. However, any heterologous protein could serve as a carrier antigen. Advantageously, a desirable carrier protein is one which is capable of enhancing the anti-carbohydrate response. Other considerations in selecting a suitable carrier protein are ease of production, solubility and coupling efficiency.

Carrier proteins which are conjugated to capsular polymers may be native toxin or a detoxified toxin also called a toxoid. In addition, genetically altered proteins which are antigenically similar to the toxin but non-toxic, called "cross reacting materials," or CRMs, may be used. $CRM_{197}$ is suitable because it has a single amino acid change from the native diphtheria toxin and is immunologically indistinguishable from it yet is nontoxic. CRMs have a significant advantage over toxoids since they do not require chemical inactivation and are thus more easily prepared.

Other carriers include toxins or toxoids of pseudomonas, staphylococcus, streptococcus, and entero toxigenic bacteria including Escherichia coli.

However, a need exists for a non-toxic, effective protein carrier for antigens containing carbohydrates which is an alternative to tetanus and diphtheria toxoids. (See Dick, W.E., Beurret, M., "Glycoconjugates of bacterial carbohydrate antigens." in J.M. Cruse & R. E. Lewis (eds.), Contributions to Microbiology and Immunology, vol. 10, Conjugate Vaccines pp 48-114 (1989).

In this regard, filamentous hemagglutinin (FHA) of Bordetella pertussis or an immunologically active portion thereof has certain advantages as a suitable carrier. As a protein, FHA is substantially nontoxic and with a molecular weight of about 200,000 KDa has many immuno-potentiating T-cell epitopes. Thus, the conjugation of the oligosaccharide to FHA provides an immunogenic conjugate which can induce or enhance the immune response to the oligosaccharide. In addition, an immune response to the coupled saccharide and the carrier protein (FHA) would enhance the protective immune response against B. pertussis. It is also important that FHA can be produced and purified on a large commercial scale.

Also suitable as carriers are outer membrane proteins (OMP), a diverse group of bacterial protein antigens known in the art. One such antigen, associated with the B. pertussis outer membrane and having an apparent molecular weight of about 69,000 daltons is referred to as the B. pertussis 69K proteins or P.69. A correlation between protection against disease and the presence of specific antibodies to the 69K protein of B. pertussis has been established. Novotny et al., Dev. Biol. Stand. 61:27-41 (1985); (Novotny et al., Infect. Immun. 50:190-198 (1985)). Others have shown that the 69K protein is protective in certain animal models of pertussis, either by active or passive immunity. Finally, the 69K protein has been reported to be a component of an acellular pertussis vaccine successfully utilized in Japan. (Aoyama et al., Am. J. Dis. Child. 143:655-659 (1989); Brennan et al., Infect. Immun. 56:3189-3195 (1988); Charles et al., Proc. Natl. Acad. Sci. USA 86:3554-3558 (1989); Leininger-Zapata et al., Abstr. Amer. Microbiol. p. 51, abstr. B-123 (1989); Shahin et al., Abstr. Amer. Soc. Microbiol. p. 51, abstr. B-125 (1989)). Thus the 69K protein of B. pertussis, being immunologically active itself, is a candidate for use as a carrier protein of the present invention.

Also within the scope of the present invention is included those carriers which consist of peptide fragments derived from or duplicative of naturally occurring bacterial peptides. Other suitable carrier proteins will be apparent to those skilled in the art. For a description of protein carrier systems, see Dick and Beurret, "Glycoconjugates of bacterial carbohydrate antigens", supra.

C. Detailed Description Of Preferred Conjugation

The conjugates of this invention can be prepared by any of the biologically compatible methods for covalently coupling antigens to carriers with the preferred carrier being FHA of Bordetella pertussis. Most of the coupling methods form either amine or amide bonds. However, thioesters can also be used.

One preferred methodology is the use of sodium cyanoborohydride ($NaBH_3CN$) following the procedures of Jennings and Lugowski, J. Immunol., 127, 1011-1018 (1981); Anderson; U.S. Patent 4,762,713 (1988); U.S. Patent 4,673,574 (1987); U.S. Patent 4,761,283 (1988).

The method of conjugation involves reacting the carrier protein such as FHA and the Bordetella pertussis oligosaccharide (BPOS) in the presence of cyanoborohydride ions, or some other reducing agent which will not reduce the reducing ends of interest nor adversely affect the carrier protein or the BPOS. The cyanoborohydrate ions (or their equivalent) act solely as a mild selective reducing agent of the Schiff base intermediate formed between the carbonyl groups of the oligosaccharide with the amino groups of the carrier protein. The carbohydrate antigen such as BPOS, is suitably prepared for conjugation by first forming reducing end groups by suitable methods which include selective hydrolysis through acids or enzymes or by oxidative cleavage using periodate. The conjugation is preferably achieved by reductive amination in an aqueous solution containing cyanoborohydride anions.

Vaccine compositions comprise the conjugates in a pharmaceutically acceptable vehicle, such as physiological saline, or ethanol polyols (such as glycerol or propylene glycol). The vaccine compositions may optionally comprise adjuvants, such as vegetable oils or emulsions thereof, surface active substances, such as hexadecylamine, octadecyl amino acid esters, octadecylamine, lysolecithin, dimethyl-dioctadecylammonium bromide, N,N-dicoctadecyl-N'-N'bis (2-hydroxyethyl-propane diamine), methoxyhexadecylglycerol, and pluronic polyols selected from polyamines, pyran, dextransulfate, poly IC, carbopol as well as peptides, such as muramyl dipeptide, dimethylglycine, and tuftsin as well as immune stimulating complexes, oil emulsions and mineral gels. The conjugates of this invention may also be incorporated into liposomes or ISCOMS (immuno-stimulating complexes). Supplementary active ingredients may also be employed. The conjugate can also be adsorbed onto a mineral suspension, such as alum, i.e., aluminum hydroxide or aluminum phosphate to further modulate the protective immune response to the antigen.

The vaccines can be administered to a human or warm-blooded animal in a variety of ways. These include intradermal, transdermal (such as by slow release polymers), intramuscular, intraperitoneal, intravenous, subcutaneous, oral and intranasal routes of administration. The amount of conjugate employed in such vaccine will vary depending upon the identity of the antigen employed. Adjustment and manipulation of established dosage ranges used with traditional carrier conjugates for adaptation to the present conjugate vaccines is well within the ability of those skilled in the art. The conjugates of the present invention are intended for use in the treatment of both immature and adult warm-blooded animals, and in particular, humans. In addition, the use of the present methods and conjugates is not limited to prophylactic application; therapeutic application are also contemplated.

The immunogenic conjugates of the invention can be admixed with an antigen from the same or different organism in a pharmaceutically acceptable vehicle and an optional adjuvant to produce a covaccine which can be used to elicit an immune response to both the conjugated antigen and the admixed antigen.

For example, the conjugate vaccine can be mixed with the other vaccine formulations, particularly diphtheria and tetanus toxoids, other pertussis components, Haemophilus influenzae type b conjugates, N. meningitidis group A and C polysaccharide or conjugate vaccines, Streptococcus pneumoniae polysaccharide or conjugate vaccines, inactivated polio and hepatitis B, measles, mumps and rubella vaccines and cytomegalovirus and herpes virus glycoproteins, singly or in combination with or without immunostimulatory agents such as cytokines (particularly interleukins 1 and 2) and/or adjuvants such as muramyl dipeptide (MDP), muramyl tripeptide (MTP) or monophosphoryl lipid A (MPLA) added to the vaccine mixture. For a description of stable vaccine compositions containing interleukins, see United States Patent application Serial Number 07/379,742 filed July 14, 1989, hereby incorporated by reference.

Other suitable antigens for use in the co-vaccine compositions of the invention include the particulate or soluble antigens from pathogenic bacteria, viruses, parasites and fungi and microcomponents of these organisms.

It is to be understood from the above discussion that the use of the term antigen is meant to imply either the whole antigen or one or more of its determinants, and is also meant to encompass hapten molecules which could be usefully mixed with the conjugates of the present invention. The foregoing list of antigens is for exemplary purposes only. Additional antigens which can be used in the co-vaccine compositions of the present invention are readily ascertained by one skilled in the art.

The invention is further illustrated by the following non-limiting Exemplification.

Example 1

(a) Bactericidal Activity of B. pertussis Anti LOS Monoclonal Antibody

The following Table 2 sets forth the bactericidal activity and dose dependent killing demonstrated by anti-LOS monoclonal antibody G10F8C3 (Li Z.M. et al., Infect. Immun. 56: 699-702 (1988)), a monoclonal antibody raised to B. pertussis LOS.

**Table 2**

**% Reduction in Bacterial cfu[1]**

| Antibody | Complement (c′) | Heat inactivated c′ |
|---|---|---|
| **G10F8C3** | | |
| 10 μg/ml | 99.5 ± 0.40 | −18.1 ± 29.6[2] |
| 1 μg/ml | 77.7 ± 3.7 | − 9.7 ± 0.7 |
| 0.1 μg/ml | 46.1 ± 4.8 | − 7.3 ± 14.9 |
| **N5-2[3]** | | |
| 10 μg/ml | 6.4 ± 17.0 | − 1.1 ± 13.1 |
| **No Ab** | 26.7 ± 4.8 | − 9.5 ± 13.8 |

1) Each reaction mixture contained approximately 100 cfu of B. pertussis Tohama I.

2) Negative number denotes an increase in bacterial cfu's.

3) Mab to a viral protein not reactive with B. pertussis

The procedures utilized in the foregoing bactericidal assay for B. pertussis were as follows:

Seventy-two hour plate cultures of Bordetella pertussis (Strain Tohama I L6) on Bordet-Gengou (BG) plates were passaged on fresh BG plates and 24 hour growths were harvested with PBS (10 mM, pH 7.4) containing 1% casamino acids. The optical density was adjusted to a Klett reading of 140-160, corresponding to a cell density of approx. $10^9$ cfu/ml. Four serial ten fold dilutions of the bacterial suspension in PBS with 1% casamino acids were made. Assays were performed in 500ul Eppendorf tubes or in sterile microtiter plate wells. Fifty microliters of PBS, 10ul 10xCa/Mg PBS (PBS with 1.5mM CaCl$_2$ and 5mM MgCl$_2$), 10ul antibody or test serum and 10ul of the diluted B. pertussis suspension were added to each tube or well. The assay mixtures were incubated at 37°C for 45 minutes and then 20ul precolostral calf serum (as the source of complement) or heat inactivated complement was added. Assay mixtures were reincubated for 45 minutes at 37°C and 10ul aliquots plated on BG agar. Colonies were counted after incubation for 48 hours at 37°C.

(b) Passive Immunization of Mice With Anti-LOS Monoclonal Antibody

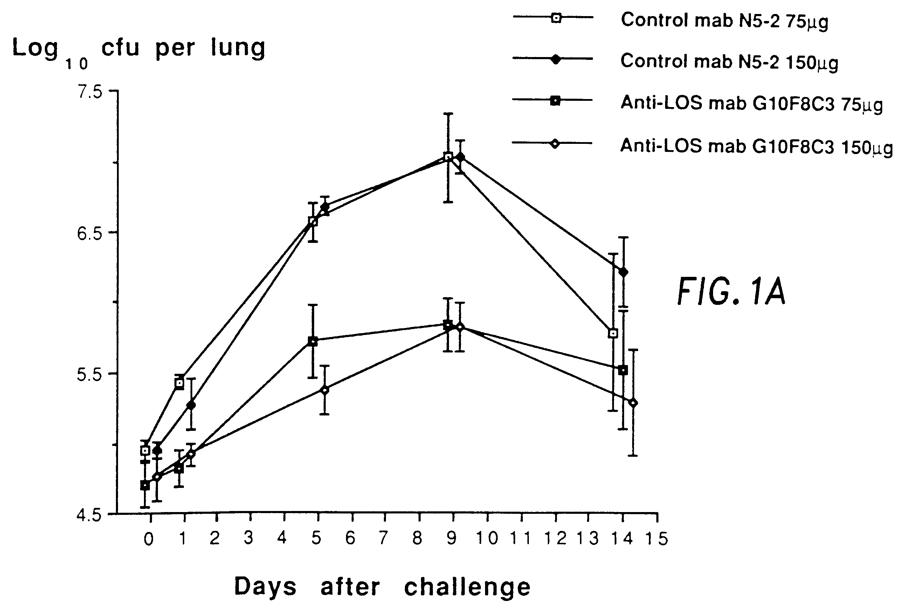

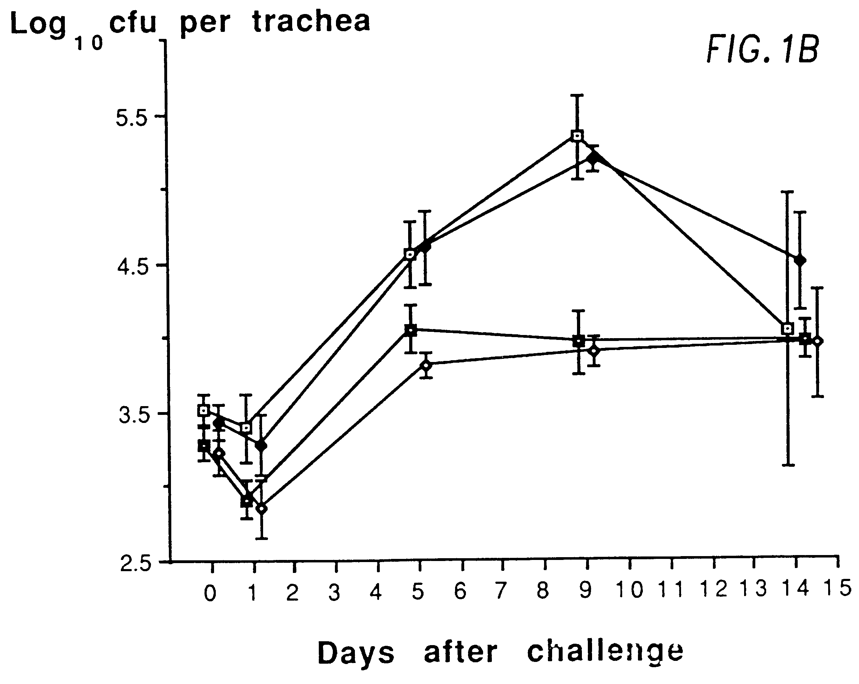

As set forth in Figure 1, the following test demonstrates that monoclonal antibodies raised to B. pertussis LOS passively protect mice from colonization with B. pertussis in vivo: Five week old Balb/c mice were passively immunized i.v. with either anti-LOS monoclonal antibody G10F8C3 or control monoclonal antibody N5-2 (a monoclonal antibody to respiratory syncytial virus F protein). Twenty-four hours later, mice were challenged with B. pertussis Tohama I in an aerosol. Mice receiving mab G10F8C3 had reduced B. pertussis colonization in both the lungs and trachea compared to mice receiving mab N5-2. Figures 1A and 1B show colonization in lungs and trachea, respectively.

Example 2

Preparation of Oligosaccharide from Bordetella pertussis Tohama I Lipopolysaccharide

Lipopolysaccharide (LPS) was purified from Bordetella pertussis Tohama I cells using the hot phenol-water extraction method as described by Johnson, K. G. and Perry, M. B. Can. J. Microbiol. 22, 29-34 (1976). The lyophilized LPS (16.1 mg) was resuspended in 1% acetic acid at a concentration of 2 mg/ml and boiled for 90 minutes. The suspension was then frozen, thawed and centrifuged at 12,000 x g for 20 minutes. The supernatant (approximately 8 ml), which contains the core oligosaccharide, was harvested and extracted with an equal volume of chloroform:methanol (3:1). The aqueous phase was then harvested and concentrated to 3.8 ml followed by further purification using gel filtration on a BioGel® (BioRad, Richmond, CA) P-4 column (200-400 mesh, 100 cm X 2.5 cm) in 0.02 M pyridinium acetate buffer pH 5.4 at a flow rate of 14 ml/hour. Fractions testing positive for hexoses by the Anthrone reaction (Scott and Melvin, 1953, Anal. Chem. 25:1656), were pooled, frozen and then lyophilized.

Example 3

Conjugation of LOS Oligosaccharide to CRM$_{197}$

Bordetella pertussis oligosaccharide (BPOS) was activated via sodium periodate (NaIO$_4$) oxidation in aqueous buffer (Jennings and Lugowski, J. Immunol., 127, 1011-8, 1981; Anderson, Infect. Immun., 39, 233-8, 1983; Anderson et al., J. Clin. Invest. 76, 52-9, 1985; Anderson, U.S. Pat. 4,761,283, 1988; Lugowski et al., J. Immunol. Methods 95, 187-94, 1986). The reaction was monitored by high performance gel permeation chromatography (HPGPC) in aqueous eluent, using ultraviolet (UV) and refractive index (RI) detection. The reaction was stopped and the activated oligosaccharide (BPOS/OX) was desalted by low pressure gel permeation chromatography (GPC) in water, and then lyophilized. A solution was then prepared in water and subsequently frozen for temporary storage. BPOS/OX and CRM$_{197}$ were mixed in aqueous neutral buffer, and the conjugation was initiated by addition of sodium cyanoborohydride (NaBH$_3$CN) (Jennings and Lugowski, J. Immunol., 127:1011-8, 1981; Anderson, U.S. Patent 4,762,713, 1988; U.S. Patent 4,673,574, 1987; U.S. Patent 4,761,283, 1988). The reaction was carried out for 4 days, while being monitored by HPGPC. The reaction was finally stopped by centrifugal diafiltration. The final preparation was stored in the cold, in the presence of thimerosal to prevent bacterial growth. The resulting glycoconjugate provides a mechanism to present the BPOS epitope to the immune system.

In preparation of the conjugate, the following experimental conditions were employed. Purified CRM$_{197}$, dissolved in 15% sucrose (at a concentration of 21.6 mg/ml), was stored at -20°C. BPOS (3.5 mg; final concentration: 2.5 mg/ml and K$_d$ = .37) was oxidized by 4 mM NaIO$_4$ in 0.05 M sodium phosphate buffer (pH 6.2-6.5) at room temperature, in the dark, with agitation. To assess the progress of the oxidation reaction, aliquots (20 $\mu$l) were withdrawn at regular intervals, the reaction stopped by addition of ethylene glycol (2

$\mu$l), and the aliquots analysed by HPGPC on Waters (Milford, MA) Ultrahydrogel™ 250 + 120 columns (2 columns coupled; 2 x 300 mm x 7.8 mm) in 0.2 M phosphate-saline buffer (PBS; 0.2 M sodium phosphate, 0.9% NaCl, pH 7.8), at a flow rate of 0.8 ml/min, using UV (206 nm) and RI detection. After 30 min, the reaction was stopped by addition of ethylene glycol (1/20 of the reaction volume), and the BPOS/OX was desalted by GPC on Bio-Rad (Richmond, CA) Bio-Gel® P-2 (200 - 400 mesh, 30 cm x 1.5 cm) in water, at 18 ml/hr. Fractions were collected (1.2 ml) and analyzed for the presence of carbohydrate (N-acetyl-hexosamine, HexNAc) (Elson and Morgan Biochem. J. 27:1824-8, 1933) and aldehydes (Porro et al., Anal. Biochem. 118:301-6, 1981). Positive fractions were pooled and lyophilized. Desalted BPOS/OX (3.3 mg) was then dissolved in water (10 mg/ml) and frozen at -20°C. Both CRM$_{197}$ ($K_d$ = 0.22) and BPOS/OX ($K_d$ = 0.37) were analyzed by HPGPC (UV at 206 and 280 nm respectively) before being frozen and prior to the conjugation. No degradation occurred during storage, as ascertained by the exact similarity of the elution profiles. BPOS/OX (2.5 mg; final concentration: 2.5 mg/ml) and CRM$_{197}$ (5 mg; final concentration: 5 mg/ml) were mixed in a polypropylene tube in 0.2 M sodium phosphate buffer (pH 7.0), and NaBH$_3$CN was added (25 $\mu$moles) to initiate the conjugation (Anderson, U.S. Patent 4,762,713, 1988; U.S. Patent 4,673,574, 1987; U.S. Patent 4,761,283, 1988). The reaction mixture was left one day at room temperature, then 3 days at 35°C, without agitation. The reaction was monitored by HPGPC (UV at 280 nm) at different stages, and finally stopped by removing NaBH$_3$CN and unreacted BPOS/OX by centrifugal diafiltration. The final preparation was analyzed for HexNAc (Elson and Morgan Biochem. J. 27:1824-8, 1933) (0.25 mg at 0.25 mg/ml) and protein (Lowry et al., J. Biol. Chem. 193:265-75, 1951) (4.4 mg at 4.4 mg/ml) content. It was then stored at 4°C in the presence of thimerosal (0.01%, w/v) to prevent bacterial growth.

Example 4

Immunoblot Analysis of Oligosaccharide - CRM$_{197}$ Conjugate

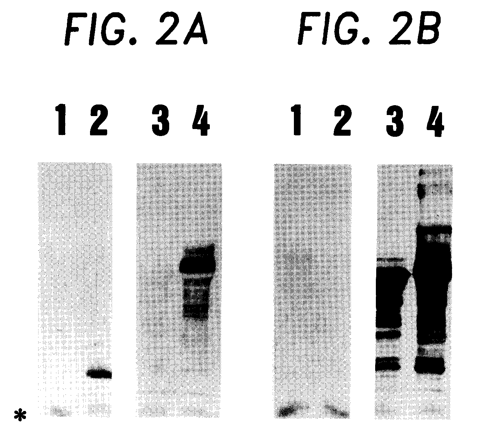

The conjugation of core oligosaccharide to CRM$_{197}$ was evaluated by immunoblot analysis. Oligosaccharide, LPS, CRM$_{197}$ and oligosaccharide-CRM$_{197}$ conjugate (5 $\mu$g each) were resolved by sodium dodecyl sulfate (SDS)-polyacrylamide gel electrophoresis (PAGE) and then transferred to nitrocellulose membranes. One set was probed with anti-LPS monoclonal antibody G10F8C3, which has been shown by Li, Z. M., et al., Infect. Immun. 56, 699-702 (1988) to be specific for the oligosaccharide of Bordetella pertussis LPS. Another set was probed with rabbit anti-CRM$_{197}$ antibody. Both the purified LPS and the core oligosaccharide-CRM$_{197}$ conjugate vaccine reacted with monoclonal antibody G10F8C3 (Figure 2, panel A, lanes 2 and 4) demonstrating covalent coupling of the oligosaccharide to CRM$_{197}$. Furthermore, this result also demonstrates that the protective epitope recognized by monoclonal antibody G10F8C3 is not compromised by the isolation and conjugation of the oligosaccharide to CRM$_{197}$. (As expected, the purified oligosaccharide was not detected with monoclonal antibody G10F8C3 [Figure 2, panels A and B, lane 1] since saccharides do not bind to nitrocellulose membranes). CRM$_{197}$, as well as the conjugate vaccine, reacted with the rabbit anti-CRM$_{197}$ antibody (Figure 2, panel B, lanes 3 and 4) indicating that the antigenicity of CRM$_{197}$ is retained following conjugation.

As discussed above, Figure 2 shows the Immunoblot analysis of Bordetella pertussis LPS oligosaccharide-CRM$_{197}$ conjugate vaccine.

Lanes 1 and 2 contain 5 $\mu$g of oligosaccharide and 5 $\mu$g of LPS, respectively (resolved by SDS-PAGE in a 15% polyacrylamide gel). Lanes 3 and 4 contain 5 $\mu$g of CRM$_{197}$ and 5 $\mu$g of oligosaccharide-CRM$_{197}$ conjugate vaccine, respectively (resolved by SDS-PAGE in a 10% polyacrylamide gel). Panel A: blot was reacted with monoclonal antibody G10F8C3 followed by goat anti-mouse immunoglobulin peroxidase conjugate. Panel B: blot is reacted with rabbit anti-CRM$_{197}$ antibody followed by reaction with goat anti-rabbit immunoglobulin peroxidase conjugate. Asterisk shows dye-front position.

Example 5

Evaluation of Immunogenicity of Oligosaccharide Conjugates

Balb/c mice were immunized subcutaneously, twice, four weeks apart with a B. pertussis oligosaccharide (B.p.OS) (10ug): CRM$_{197}$ (395ug) conjugate or an admixture of B.p.OS (10 ug) + CRM$_{197}$ (395 ug). Monophosphoryl-lipid A, MPL A (40ug) or aluminum phosphate, alum (200ug) were used as adjuvant. Mice were bled at weeks 0, 4, 6 and 8. Sera were assayed for anti-LOS antibodies by ELISA and week 8 sera were assayed for bactericidal activity against B.p. strain Tohama L6.

ELISA ASSAY FOR ANTI-B. PERTUSSIS LOS ANTIBODIES

Mouse anti-LOS antibody was measured by the following procedure:

LOS from B. pertussis Tohama L6 was diluted to 20ug/ml in sterile PBS and used to coat the wells of NUNC (NUNC, Denmark) polysorb plates overnight at 37°C. Buffer and unadsorbed coating antigen were shaken out and wells blocked with sterile PBS containing 0.1% Gelatin for 1 hour at 37°C.

Appropriate dilutions of serum or antibodies, 100ul in PBS, 0.05% Triton X-100 were pipetted into the LOS coated and blocked plate wells and incubated at 37°C for 1 hour. Plates were washed with PBS, 0.1% Triton X-100 and bound mouse IgG incubated with a 1:500 dilution of Goat anti-mouse IgG + IgM.Alkaline phosphatase conjugate(Tago, Burlingame, CA) for 1 hour at 37°C, washed with PBS, 0.1% Triton X-100 and developed with 100ul p-nitrophenylphosphate : Sigma 104 Alkaline Phosphatase substrate, 1mg/ml (Sigma Chemical Co., St. Louis, MO.) at room temperature for 1 hour. The reaction was stopped with 50ul 3N NaOH in each well.

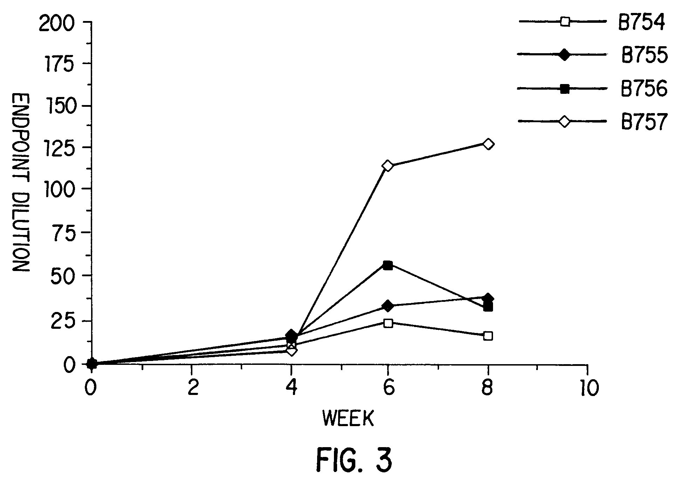

The optical density developed was read at 405nm and 690nm on a Biotek ELISA plate reader Model EL310 and data from both wavelengths used to plot endpoint dilutions. The results are set forth in Figure 3.

Legend for Figure 3 and Table 3:

B754: Mixture of B.p.OS and CRM$_{197}$ with Alum
B755: Mixture of B.p.OS and CRM$_{197}$ with MPL A
B756: OS-CRM$_{197}$ Conjugate with Alum
B757: OS-CRM$_{197}$ Conjugate with MPL A

BACTERICIDAL ACTIVITY

## Table 3

## % reduction of viable B.p. Tohama L6 by sera

| Sample | Assay 1 | Assay 2 |
|---|---|---|
| B754 1:10 diln | 5.4 | 5.7 |
| B755 1:10 diln | 11.7 | 11.5 |
| B756 1:10 diln | 28.5 | 32.8 |
| B757 1:10 diln | 32.8 | 39.5 |
| No Antibody | 6.8 | 10.1 |
| Control serum* | N.D. | 99.5 |

*Assay control serum (100ug/ml IgG) contains anti-LOS antibodies and was raised against an FHA preparation contaminated with B.p.LOS. This antiserum was previously assayed and shown to have bactericidal activity. The anti-LOS monoclonal antibody G10F8C3 did not show any detectable bactericidal activity in these assays.

**Claims**

1. An immunogenic conjugate comprising an oligosaccharide or fragment thereof derived from the lipooligosaccharide of Bordetella pertussis, having one or more antigenic determinants of the endotoxin of Bordetella pertussis conjugated to a carrier protein or peptide.

2. An immunogenic conjugate according to Claim 1, wherein the carrier protein or peptide is $CSM_{197}$ or a peptide derived from $CRM_{197}$.

3. An immunogenic conjugate according to Claim 1, wherein the carrier protein or peptide is FHA of Bordetella pertussis or a peptide derived therefrom.

4. An immunogenic conjugate according to claim 1 wherein the carrier protein or peptide is a bacterial outer membrane protein or peptide derived therefrom.

5. A vaccine composition comprising an immunogenic conjugate of an oligosaccharide or fragment thereof derived from the lipooligosaccharide of Bordetella pertussis having one or more antigenic determinants of the endotoxin of Bordetella pertussis coupled to a suitable carrier protein or peptide and a pharmaceutically acceptable vehicle.

6. A vaccine composition according to Claim 6, wherein the suitable carrier protein or peptide is $CRM_{197}$ or a peptide derived therefrom.

7. A vaccine according to Claim 6, wherein the suitable carrier protein or peptide is FHA of Bordetella pertussis or a peptide derived therefrom.

8. A vaccine composition of claim 6 further comprising an adjuvant.

9. A co-vaccine composition comprising an immunogenic conjugate according to claim 1 in combination with one or more additional antigenic and/or immunostimulatory agents in a pharmaceutically acceptable vehicle and an optional adjuvant.

10. A method of eliciting an immune response against Bordetella pertussis comprising administering to a warm-blooded animal an effective amount of a vaccine composition comprising a conjugate which is an oligosaccharide or fragment thereof derived from Bordetella pertussis lipooligosaccharide coupled to a carrier protein or peptide and a pharmaceutically acceptable vehicle.

Log$_{10}$ cfu per lung

Legend:
- □ Control mab N5-2 75μg
- ◆ Control mab N5-2 150μg
- ■ Anti-LOS mab G10F8C3 75μg
- ◇ Anti-LOS mab G10F8C3 150μg

*FIG.1A*

Days after challenge

EP 0 471 954 A2

**Log$_{10}$cfu per trachea**

*FIG.1B*

**Days after challenge**

EP 0 471 954 A2

# FIG. 2A    FIG. 2B

## 1 2  3 4    1 2  3 4

FIG. 3

EP 0 471 954 A2